# EUROPEAN PATENT APPLICATION

(11) **EP 4 846 970 A2**
(43) Date of publication of application: **02.12.2026**
(21) Application number: 26198500.6
(22) Date of filing: 28.09.2023
(51) Int. Cl.: A61B 34/00

(54) **DISPLAY FOR ELECTRODE PROXIMITY**

(30) Priority: 03.10.2022 US 202263412760 P
(62) Document number(s) of the earlier application(s) in accordance with Art. 76 EPC: 23797945.5 / 4 598 440
(71) Applicant: St. Jude Medical, Cardiology Division, Inc., St. Paul, MN 55117 (US)
(72) Inventor: BULLEMER, Beth C., Saint Paul, Minnesota, 55105 (US); BETZLER, Eric J., Andover, Minnesota, 55304 (US); RUETZ, Linda, New Brighton, Minnesota, 55112 (US); SCHWEITZER, Jeffrey A., Saint Paul, Minnesota, 55108 (US); VOTH, Eric J, Maplewood, Minnesota, 55109 (US); GARFIELD, M. Robert, Cincinnati, Ohio, 45242 (US)
(74) Representative: Mathys & Squire

(57) **Abstract**

A display system configured to communicate tissue proximity data from a plurality of electrodes includes a display. The display outputs a circular graphical representation. The circular graphical representation includes a plurality of color areas forming a first circular shape. Each of the color areas communicate a categorized value for a respective electrode indicative of tissue proximity for the respective electrode. A plurality of graph areas form a second circular shape. Each of the graph areas communicate a relative value for the respective electrode indicative of the tissue proximity for the respective electrode.

## Description

### CROSS-REFERENCE TO RELATED APPLICATION

This application claims benefit of priority to U.S. Provisional Patent Application No. 63/412,760 filed on October 3, 2022, the entire disclosure of each of which is hereby incorporated by reference.

### BACKGROUND

The present invention relates generally to catheters, and methods and systems of detecting the proximity of tissue to electrodes based on electrical impedances.

Catheters are utilized in a number of operations within the human body. In many of these applications, whether collecting data from surrounding tissue or administering treatment, it is important to determine whether portions of the catheter - in particular the electrodes collecting data and/or administering treatment - are in contact with, or in close proximity to, the adjacent tissue. A number of methods are utilized to make this determination, including for example monitoring electrocardiogram signals (e.g., voltage measured between electrodes) and/or impedance of an electrode. For example, impedance is understood, in general, to increase in response to contact with tissue. However, a number of other factors may also result in variations in impedance, including location of the electrode within the body (i.e., different chambers of the heart, exposed to different volumes of blood flow, may exhibit different impedance values) and movement of the surrounding tissue as a result of, for example, heartbeats. These factors make it difficult to rely on impedance measurements. It would therefore be beneficial to develop a method of reliably detecting proximity based on impedance measurements.

Further, the efficient communication of data in a clinical workflow is important to improve efficacy of treatment, minimize errors, and reduce procedure time. It would therefore be beneficial to develop a method of displaying electrode contact and/or electrode proximity in an effective manner.

### SUMMARY

Embodiments of the present disclosure describe a display system configured to communicate tissue proximity data from a plurality of electrodes, the display system comprising a display configured to output a circular graphical representation, the circular graphical representation including a plurality of color areas forming a first circular shape, each of the color areas configured to communicate a categorized value for a respective electrode indicative of tissue proximity for the respective electrode, and a plurality of graph areas forming a second circular shape, each of the graph areas configured to communicate a relative value for the respective electrode indicative of the tissue proximity for the respective electrode.

Embodiments of the present disclosure describe a user interface system for a catheter device, the user interface system comprising a display, including a circular proximity display area, the circular proximity display area including a plurality of circular sectors, each of the circular sectors corresponding to one or more electrodes disposed on the catheter device, and one or more control features configured to manipulate the catheter device and/or the electrodes disposed on the catheter device, wherein the one or more control features are configured to select one or more system states.

Embodiments of the present disclosure describe a method of display, comprising receiving tissue proximity data from a plurality of electrodes, transforming received data into a circular arrangement, the circular arrangement including a plurality of circular sectors, each of the circular sectors corresponding to one or more electrodes, and displaying the circular arrangement on a user interface, wherein each of the circular sectors includes a categorized value display and a relative value display.

The details of one or more examples are set forth in the description below. Other features, objects, and advantages will be apparent from the description and from the claims.

### BRIEF DESCRIPTION OF DRAWINGS

This written disclosure describes illustrative embodiments that are nonlimiting and non-exhaustive. In the drawings, which are not necessarily drawn to scale, like numerals describe substantially similar components throughout the several views. Like numerals having different letter suffixes represent different instances of substantially similar components. The drawings illustrate generally, by way of example, but not by way of limitation, various embodiments discussed in the present document.

Reference is made to illustrative embodiments that are depicted in the figures, in which:
**FIG. 1** is a side view of a medical device, according to one or more embodiments of the present disclosure.
**FIG. 2** is an expanded side view of a medical device, according to one or more embodiments of the present disclosure.
**FIG. 3** is an isometric view of a medical device, according to one or more embodiments of the present disclosure.
**FIG. 4** is an isometric view of a medical device, according to one or more embodiments of the present disclosure.
**FIG. 5** is an exemplary embodiment of a display in a tissue proximity mode, according to one or more embodiments of the present disclosure.
**FIG. 6** is an exemplary embodiment of a display in a baseline collection mode, according to one or more embodiments of the present disclosure.
**FIG. 7** is an exemplary embodiment of a 2 dimensional (2-D) plot display, according to one or more embodiments of the present disclosure.
**FIG. 8** is a flow chart of system state progression, according to one or more embodiments of the present disclosure.
**FIG. 9** is an exemplary embodiment of a display in a disabled system state, according to one or more embodiments of the present disclosure.
**FIG. 10A** is an exemplary embodiment of a display in an electrode selection system state, according to one or more embodiments of the present disclosure.
**FIG. 10B** is an exemplary embodiment of a display in an electrode selection system state, according to one or more embodiments of the present disclosure.
**FIG. 10C** is an exemplary embodiment of a display in an electrode selection system state, according to one or more embodiments of the present disclosure.
**FIG. 10D** is an exemplary embodiment of a display in an electrode selection system state, according to one or more embodiments of the present disclosure.
**FIG. 11A** is an exemplary embodiment of a display in a baseline collection system state, according to one or more embodiments of the present disclosure.
**FIG. 11B** is an exemplary embodiment of a display in a baseline collection system state, according to one or more embodiments of the present disclosure.
**FIG. 11C** is an exemplary embodiment of a display in a baseline collection system state, according to one or more embodiments of the present disclosure.
**FIG. 12A** is an exemplary embodiment of a display in a tissue proximity system state, according to one or more embodiments of the present disclosure.
**FIG. 12B** is an exemplary embodiment of a display in a tissue proximity system state, according to one or more embodiments of the present disclosure.
**FIG. 12C** is an exemplary embodiment of a display in a tissue proximity system state, according to one or more embodiments of the present disclosure.
**FIG. 13A** is an exemplary embodiment of a display in a therapy delivery system state, according to one or more embodiments of the present disclosure.
**FIG. 13B** is an exemplary embodiment of a display in a therapy delivery system state, according to one or more embodiments of the present disclosure.
**FIG. 14** is a flow chart of a method of display, according to one or more embodiments of the present disclosure.

### DETAILED DESCRIPTION

According to some embodiments, the claimed invention utilizes a circular shaped graphical representation on a display to reflect the shape of a catheter. The use of a representative shape (as opposed to a linear graphical representation) supports the transformation and interpretation of the information shown on the display (which is a representation of reality) to infer more easily what is physically happening, but not visible with the catheter. Likewise, the display behavior within the tissue proximity mode creates a visual effect supporting the visualization of catheter manipulation.

According to some embodiments, the claimed invention presents tissue proximity information at various levels of fidelity. For instance, color segments may be used to show categorized value, i.e., optimal proximity, sub-optimal proximity, as well as electrodes in an error state and deselected therapeutic electrodes. In addition to reinforcing the categorized value, an inner graph area may identify the placement of the value within the category/range (i.e., the relative value). Such an approach facilitates information processing at different levels.

In some embodiments, the claimed invention utilizes direct manipulation to select active therapeutic electrodes. Direct manipulation is an interaction style in which users act on displayed objects of interest using physical, incremental, reversible actions whose effects are immediately visible on the screen. Rather than having a separate controller to control the selection of an electrode, an operator may select an electrode directly through interacting with the graphical representation displayed on the display device. And according to some embodiments of the present disclosure, the claimed invention integrates system states that gatekeep functionality. For instance, the display appearance may change to direct an operator's attention to the recommended action or viable next step.

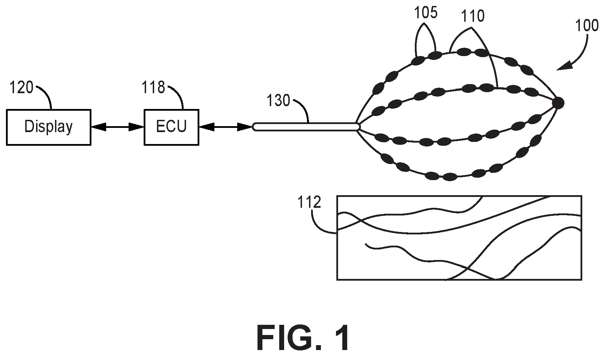

FIGS. 1-4 illustrate an exemplary medical device 100, such as a diagnostic and/or therapeutic catheter, a sheath, or other similar type of device. The medical device 100 includes a distal end and a proximal end (not shown) that includes a handle (not shown) operated by a technician and a connection point (not shown) to interface the medical device with an electronic control unit (ECU) 118 and a display 120. The distal end of the medical device may include a plurality of electrodes 105 or electrode pairs 105a and 105b.

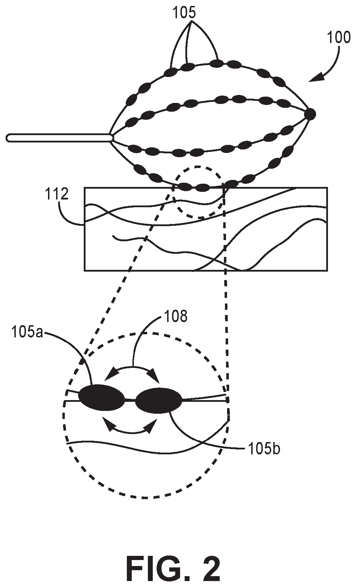

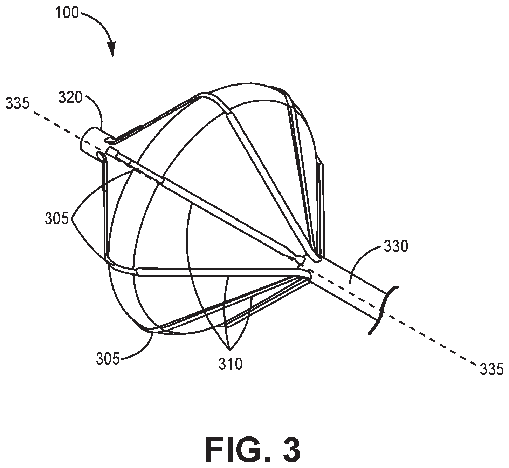

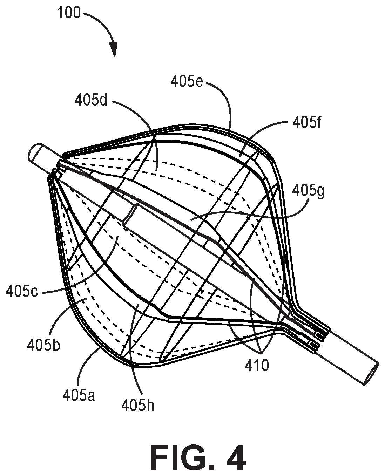

As shown in FIGS. 1-2, the medical device 100 may include a plurality of splines 110 extending from the medical device 100, wherein each of the splines 110 includes one or more electrodes 105, or as illustrated in FIG. 2, an electrode pair 105a-105b. In the embodiment shown in FIGS. 1 and 2, each of the splines 110 includes a plurality of electrodes 105, with FIG. 2 illustrating a voltage and/or current 108 measured between the electrodes 105a-105b. In other embodiments, such as that shown in FIGS. 3 and 4, each of the splines 310 and 410 may include a single electrode region 305 and 405a-405h, respectively. Each of the electrode regions 305 and 405a-405h may include one or more electrodes. In some embodiments, the medical device may include eight splines (see e.g., splines 310, 410 in FIGS. 3-4). In other embodiments, any number of splines 110, 310, 410 may be used.

As shown in FIG. 3, the medical device 100 includes a distal end 320 and a central wire 330 defining a central axis 335, with the plurality of splines 310, each of the plurality of splines 310 having an electrode region 305, disposed radially outward from the central axis 335. In some embodiments, the electrode region 305 is disposed on the portion of the spline 310 farthest from the central axis 335. Stated differently, the electrode region 305 may be disposed on the radially outermost portion of the spline 310 with respect to the central axis 335. In some embodiments, the electrode regions 305 and/or the plurality of splines 310 may deliver an ablation treatment to a target tissue.

As shown in FIG. 4, the medical device 100 may include a plurality of electrode regions 405a-405h, each electrode region corresponding to a spline 410 disposed on the medical device 100. In some embodiments, each of the electrode regions 405a-405h may include an electrode pair, wherein a voltage, impedance, current, or other electrical signal is measured across the electrode pair. In other embodiments, each of the electrode regions 405a-405h may include a single electrode. In some embodiments, a voltage, impedance, current, or other electrical signal may be measured across corresponding electrode regions 405a-405h. For example, a voltage, impedance, current, or other electrical signal may be measured across the electrode region 405a and the electrode region 405b. In some embodiments, the electrode regions 405a-405h and/or the plurality of splines 410 may deliver an ablation treatment to a target tissue. FIGS. 1-4 illustrate a circular distribution of electrodes/splines disposed circumferentially around a central axis. In some embodiments, the medical device 100 may include different arrangements of electrodes/splines, including for example, a grid or array of electrodes on a single plane, one or more loops of electrodes, electrodes disposed along a single elongate member, and/or other configurations of electrodes/splines.

With respect to the embodiments shown in FIGS. 1-4, proximity of the plurality of electrodes 105 (or electrodes 305, 405) located at the distal end of the medical device 100 may be determined based on bipolar electrode complex impedance (BECI) measurements. In general, BECI measurements are generated by driving an excitation signal between two electrodes forming a bipolar pair. The resulting voltage at each of the electrodes is measured and utilized to derive a complex impedance signal. The ECU 118 utilizes the measured BECI measurements to determine contact and/or proximity status of each electrode with respect to the adjacent tissue 112.

Determining contact status of electrodes 105 may include a tissue proximity mode, wherein a baseline measurement is used to convert real-time impedance measurements for each of the plurality of electrodes 105 into a tissue proximity score associated with electrode to cardiac tissue proximity. In general, the tissue proximity score is the ratio of the real-time impedance measurement over the baseline value. The tissue proximity score may be compared to fixed, device specific (or procedure specific) thresholds used to assign a tissue proximity category to each electrode (i.e., optimal/in contact, suboptimal/not in contact).

In some embodiments, an accurate baseline measurement may be required to calculate a reliable tissue proximity measurement, and thus, determining contact status of the electrodes 105 may include a baseline collection mode. The baseline collection mode may help the user identify when an acceptable baseline measurement has been collected for each of the electrodes 105. The baseline collection mode may include a progression of conditions, wherein each condition satisfied increases the reliability of the baseline value.

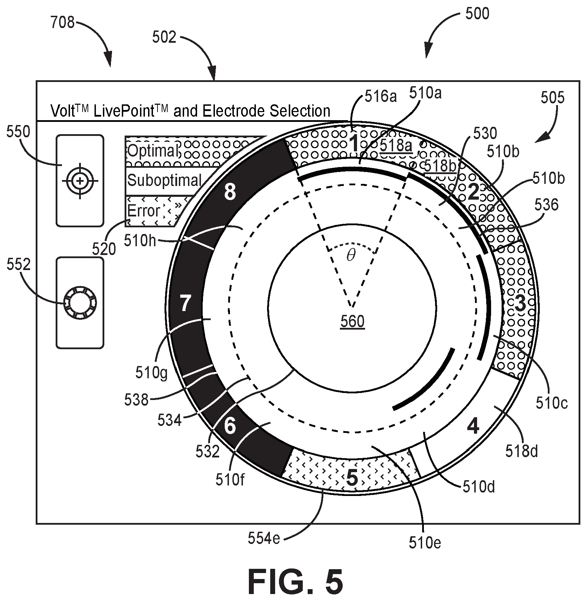

FIGS. 5-14 illustrate devices, systems, and methods of displaying impedance-based measurements, such as the impedance-based measurements described above, and manipulating medical devices through a display user interface. In the embodiments shown in FIGS. 5-14, a display 500 outputs a circular graphical representation of electrode proximity data to a user in real-time. The display 500 reflects the high-level needs of the user, resulting in a simplified display that conveys meaningful information in an efficient and accurate manner.

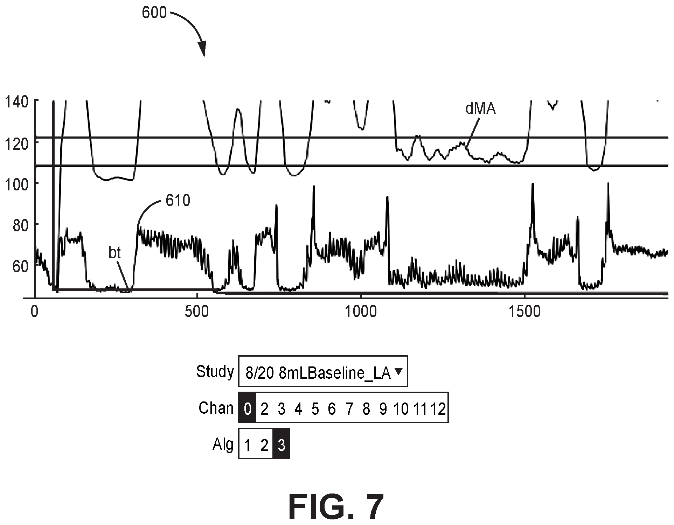

Raw BECI data may be difficult to analyze in real-time during a cardiac procedure. For example, FIG. 7 illustrates an exemplary embodiment of a 2-dimensional (2-D) plot 600 of a raw BECI baseline measurement 610. Each electrode and/or electrode pair on a catheter device (for example, the medical device 100 as shown in FIGS. 1-4), transmits data back to the ECU 118 which is displayed on the 2-D plot 600, resulting in a number of different plotlines. The 2-D plot of baseline measurement 600 is inefficient, as the user must decipher, in real-time, what each plotline represents, which electrode is selected, the x-axis and y-axis scale and/or units of measurement, etc. Therefore, there exists a need for a display system that minimizes unnecessary information and presents meaningful measurement data to improve efficiency and facilitate clinical workflow.

As shown in FIG. 5, the display 500 includes a circular display area 505 and a plurality of circle sectors 510a-510h. In the embodiment shown in FIG. 5, each of the eight circle sectors 510a-510h (collectively referred to as the circle sectors 510) correspond to an electrode and/or spline on a catheter device (for example, the electrodes 405a-405h and/or splines 410 in FIG. 4). In some embodiments, each of the circle sectors 510 may correspond to an electrode, an electrode pair, or a spline on a catheter device. The catheter device 100 may include any number of splines 110, 310, 410, and thus, the number of the circle sectors 510 may change depending on the number of splines 110, 310, 410 included in the connected catheter device 100.

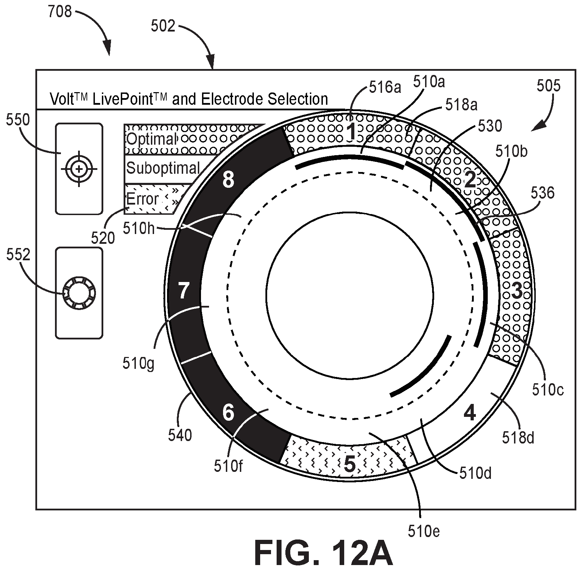

FIG. 5 illustrates the display 500 in a tissue proximity system state (see also FIGS. 12A-12C). For simplicity, only the circle sectors 510a and 510b are fully labeled. Each of the circle sectors 510a-510h include an electrode label 516a-516h, in this case, the electrode labels 516a-516h (collectively referred to as the electrode labels 516) are numbered "1" through "8". Each of the circle sectors 510a-510h may further include a color area 518a-518h (collectively referred to as the color areas 518), the color areas 518a-518h indicating a categorized value of each electrode and/or electrode pair. For example, a first color (e.g., blue) may be utilized to indicate optimal contact/proximity between a particular electrode and adjacent tissue, a second color (e.g., white) may be utilized to indicate sub-optimal contact/proximity, and a third color (e.g., yellow) may be utilized to indicate an error state. In other embodiments, additional colors may be utilized to indicate additional proximity/contact status or operating states of electrodes. As shown in FIGS. 5-6, the display 500 may include a color table area 520 to communicate what categorized value each respective color represents.

The display of a categorized value (represented by a color) is beneficial, as it allows for the efficient communication of information without overwhelming the user. The specific numerical baseline values or numerical proximity values may be irrelevant to the user, and instead, the user only needs to know whether a certain threshold is met. In such cases, displaying a number or numerical representation (e.g., a bar graph or 2-D plot) is not the most efficient form of commination, as the user is forced to compare a numerical value/numerical representation to a threshold value and determine whether said threshold is met. In contrast, the display 500 communicates whether electrodes corresponding to each of the circle sectors 510a-510h have satisfied a threshold requirement without any further steps by the user.

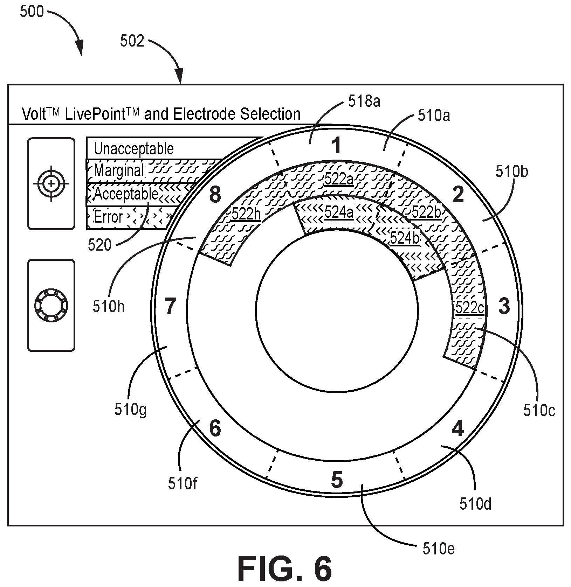

In some embodiments, the color areas 518a-518h and the color table area 520 may change from system state to system state. For example, FIG. 5 illustrates the display 500 in the tissue proximity system state, with the color area 518 limited to the outermost ring of each of the circle sectors 510, and the color table area 520 displaying a first color, a second color, and a third color. In contrast, FIG. 6 illustrates the display 500 in the baseline collection system state, with the color area 518 extending radially inward into a second ring section 522 and a third ring section 524, the color table area of FIG. 6 displaying a second color, a third color, a fourth color, and a fifth color. The use of different color schemes for different system states may minimize user error, as for example, a green color immediately communicates to the user that the system is in a baseline collection state and not in a tissue proximity state.

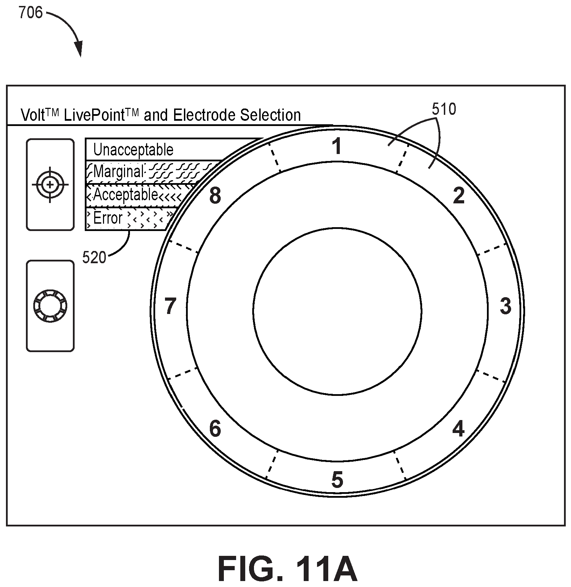

As shown in FIG. 5, each of the circle sectors 510a-510h may include a graph section 530 that extends radially inward from the color areas 518a-518h, the graph section 530 configured to communicate a relative value of an electrode measurement. The graph section 530 may include an inner boundary line 532, a threshold line 534, a current value bar 536, and an outer boundary line 538. The threshold line 534 may be generated based on the baseline collection system state (see FIGS. 6 and 11A-11C), and/or be based on fixed threshold values programmed into the system. For instance, the threshold line 534 may be generated by collecting baseline data in the baseline collection state and setting a fixed threshold value/ratio above the baseline (for example, 20% above baseline). The fixed threshold value above the baseline may vary, but typically ranges between 1% and 100% above the baseline value. In other embodiments, the fixed threshold value above the baseline may be greater than 100% above baseline. The threshold line 534 may represent the boundary threshold between an optimal proximity value and a suboptimal proximity value. In the embodiment shown in FIG. 5, the threshold line 534 is consistent between the circle sectors 510a-510h. The current value bar 536 may represent a real-time tissue proximity value of a respective electrode or electrode pair, wherein an optimal tissue proximity value would be represented by the current value bar 536 disposed radially outward from the threshold line 534 (see for example, the circle sectors 510a-510c), and a sub-optimal tissue proximity value would be represented by the current value bar 536 disposed radially inward from the threshold line 534 (see for example, the circle sector 510d). In other words, as the tissue proximity value increases (indicating the electrode is in close proximity to the tissue), the current value bar 536 is pushed radially outward to reflect the tissue proximity value relative to the threshold line 534. In the embodiment shown in FIG. 5, when the current value bar 536 is disposed radially outward from the threshold line 534 (see for example, the circle sectors 510a-510c), the respective color area(s) may display a first color indicative of an optimal electrode proximity. A relative value display is beneficial, as the user can view how close, or how far, each electrode measurement is to the optimal tissue proximity value.

The display 500 may output a circular display area 505 having a circular shape to give the user a visual representation of the circular catheter with the electrodes distributed around the circumference of the circular catheter. The use of a representative circular shape (as opposed to a linear display) supports the transformation and interpretation of information shown on the display, to help the user infer what is physically happening to the catheter device. In some embodiments, the plurality of color areas 518a-518h fit together to form a first circular shape, and the plurality of graph areas 530a-530h fit together to form a second circular shape. The circular display area 505, the first circular shape, the second circular shape, and the center circular display 560 form a plurality of concentric circles to efficiently communicate tissue proximity data.

In some embodiments, each of the circle sectors 510a-510h includes an equal outer arc length *L* and an equal central angle *θ*. For instance, the display 500 illustrates eight circle sectors 510a-510h corresponding to eight electrodes or electrode pairs (for example, corresponding to electrodes 405a-405h), each of the circle sectors 510a-510h having an outer arc length *c*/*8* (*L=c*/*8*) where "*c*" is the circumference of the circular display area 505, and each of the circle sectors 510a-510h having a central angle of *45*° (*θ= 360°*/*8*). In other embodiments, a different number of circle sectors may be used to correspond to a different number of electrodes and/or splines.

The display 500 includes a display user interface 502, which may include one or more control features, e.g., a baseline mode control 550, a select all electrodes control 552, selectable individual electrode controls 554a-554h, a proximity mode control (not shown), and a treatment mode control (not shown). The one or more control features may be selectable through the display user interface 502, and the one or more control features may control functions of the treatment device (for example, the device 100 in FIGS. 1-4) and/or may control electrode measurement functions. For example, the one or more control features may include a selectable electrode feature wherein an individual electrode and/or electrode pair may be selected for impedance-based measurements and/or to activate or deactivate therapy delivery capability of the electrode. In some embodiments, electrodes may be deselected. The deselection of electrodes may be beneficial to help direct the user's attention to the electrodes associated with therapy delivery. In some embodiments, the selection or deselection of electrodes will only impact whether data is shown to the user, i.e., electrode measurements will proceed regardless of a selected or deselected state. The deselection of an electrode may selectively disable the electrode from therapy delivery. In some embodiments, the display 500 may integrate one or more system states into the display user interface 502.

The one or more control features may be configured to allow a direct manipulation of the catheter device. The term "direct manipulation" is an interaction style in which users act on displayed objects of interest using physical, incremental, reversible actions whose effects are immediately visible on the screen. Rather than having a separate controller to control the selection of an electrode, an operator may select an electrode directly through manipulating the display device. In some embodiments, the user may interact with the display user interface 502 via a touch screen, a mouse/keyboard, and/or other buttons/controls known in the art.

FIG. 6 illustrates an exemplary embodiment of the display 500 in a baseline collection mode (see also FIGS. 11A-11C). The display 500 includes the circle sectors 510a-510h and the color areas 518a-518h. The embodiment in FIG. 6 further includes the circle sectors 510a-501h having a first ring section 522 (see first ring sections 522a-522c, 522h corresponding to the circle sectors 510a-510c, 510h) and a second ring section 524 (see second ring sections 524a, 524b corresponding to the circle sectors 510a, 510b), wherein the first ring section 522 indicates a marginal baseline collection from a respective electrode and the second ring section 524 indicates an acceptable baseline collection from a respective electrode. The first ring section 522 may have a fourth color (e.g., light green) when the corresponding electrode collects a marginal baseline measurement, and the second ring section 524 may have a fifth color (e.g., dark green) when the corresponding electrode collects an acceptable baseline measurement. The first ring section 522 and the second ring section 524 of the circle sectors 510a-510h corresponding to electrodes that have not collected a marginal or acceptable baseline measurement (for example, the circle sectors 510d-510f) may remain empty.

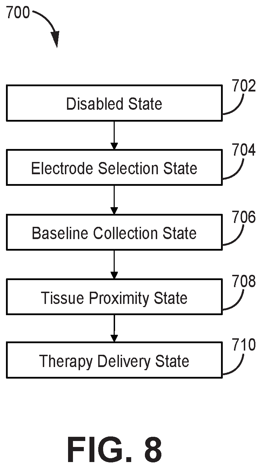

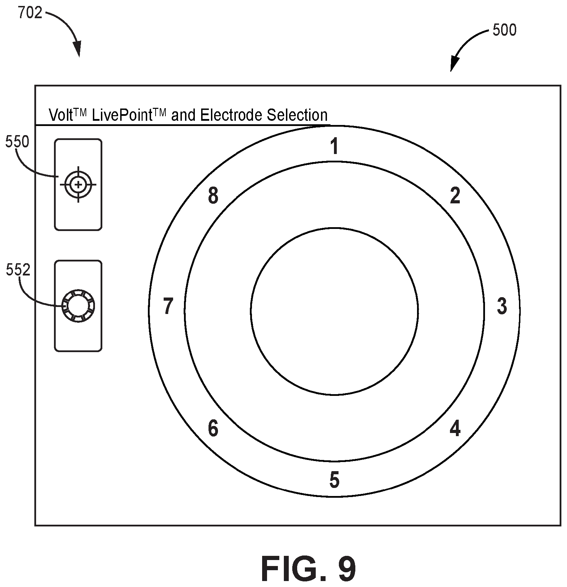

As shown in FIG. 8, a flow chart 700 of an exemplary progression of system states is shown. The display 500 having the display user interface 502 may progress through a disabled state 702, an electrode selection state 704, a baseline collection state 706, a tissue proximity state 708, and/or a therapy delivery state 710. FIGS. 9-13B illustrate exemplary displays 500 of each of the plurality of system states 702, 704, 706, 708, and 710. In some embodiments, the progression of system states 702, 704, 706, 708, and 710 is not linear. For instance, the user may go back-and-forth between the tissue proximity state 708 and the baseline collection state 706.

The disabled state 702 indicates that the catheter connection has not been established. FIG. 9 illustrates an exemplary embodiment of the display 500 in the disabled state 702. As shown in FIG. 9, the baseline mode control 550 and the proximity mode control 552 are disabled. In some embodiments a dark grey coloring is utilized to indicate the disabled state 702. In the disabled state 702, the user cannot access the one or more control features and the display 500 shows no data. Once a catheter connection is established, the display 500 and display user interface 502 may proceed to an electrode selection state 704.

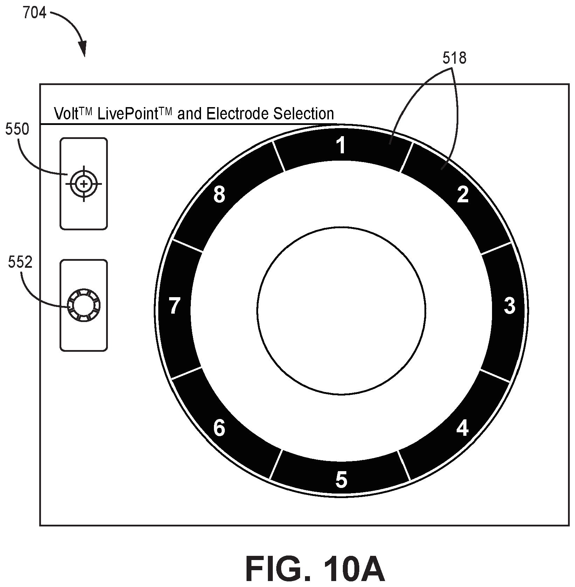

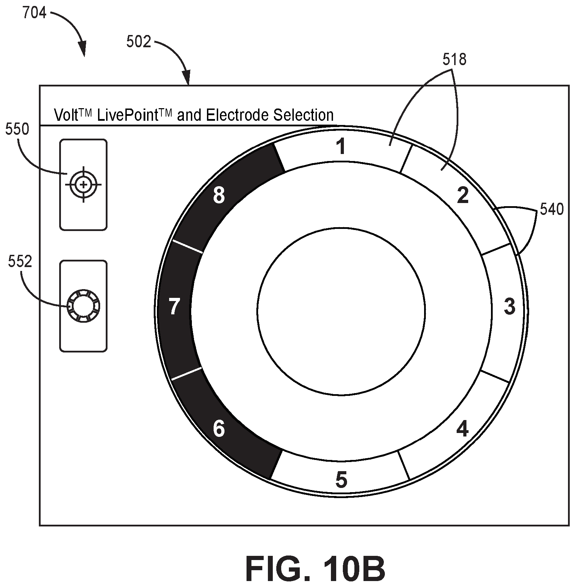

An exemplary embodiment of the electrode selection state 704 is shown in FIGS. 10A-10D. As shown in FIG. 10A, the color areas 518 corresponding to the electrode labels 516a-516h may be assigned a first color (e.g., dark grey), indicating that the respective electrodes are inactive. However, as shown in FIG. 10B, the color areas 518 corresponding to the electrode labels 516a-516e may be assigned a second color (e.g., white), indicating that the respective electrodes are active. The color areas 518 may include an outline area 540 to indicate that the respective electrode is selectable. Selecting an electrode may be achieved through the display user interface 502. Selection of an electrode may enable the electrode to deliver a therapy treatment and/or display electrode proximity data on the display 500. Deselection of an electrode may disable therapy delivery of the electrode and/or disable the display of electrode proximity data on the display 500. The selection and/or deselection of electrodes may be beneficial, as in some cases, some of the electrodes on the medical device 100 may have incidental contact with, or be within close proximity to, the esophagus, nerves, and/or other non-target tissue. Thus, delivering an ablation treatment to all electrodes may result in incidental damage to non-target tissue. In some embodiments, the electrodes may be selected and/or deselected to deliver different magnitudes of treatment at each electrode. For example, a first electrode may be selected and a first treatment may be delivered, and subsequently, a second electrode may be selected and a second treatment may be delivered.

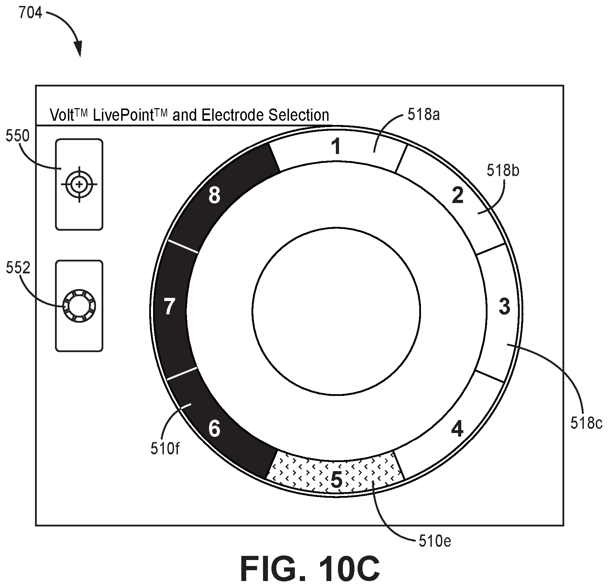

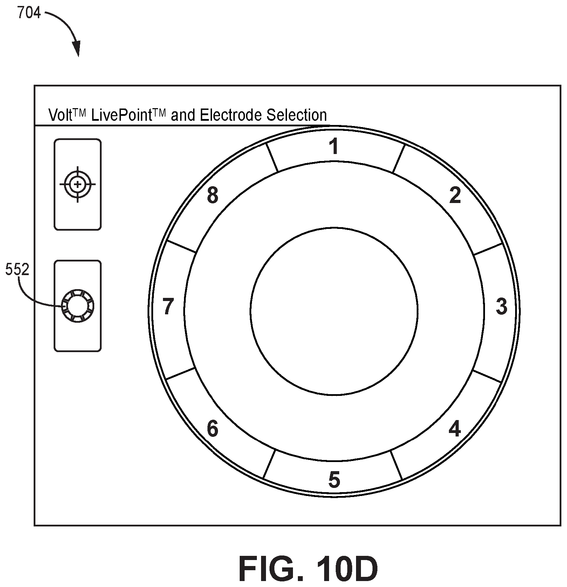

FIG. 10C illustrates an exemplary embodiment wherein the circle sector 510e displays an error. In other words, the electrode and/or spline corresponding to the circle sector 510e may be damaged, disconnected, or otherwise malfunctioning, and thus, the user is notified of the error prior to the collection of electrophysiology data. FIG. 10D illustrates an exemplary embodiment where all electrodes are selected and operational. The user may select the baseline mode control 550 in the display user interface 502 to begin the baseline collection state 706.

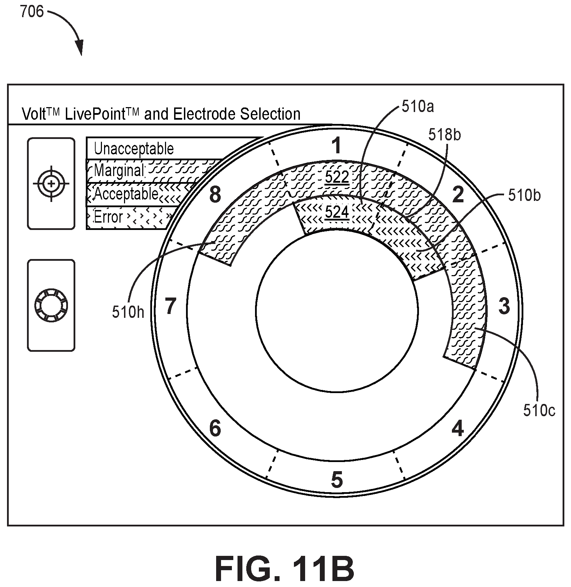

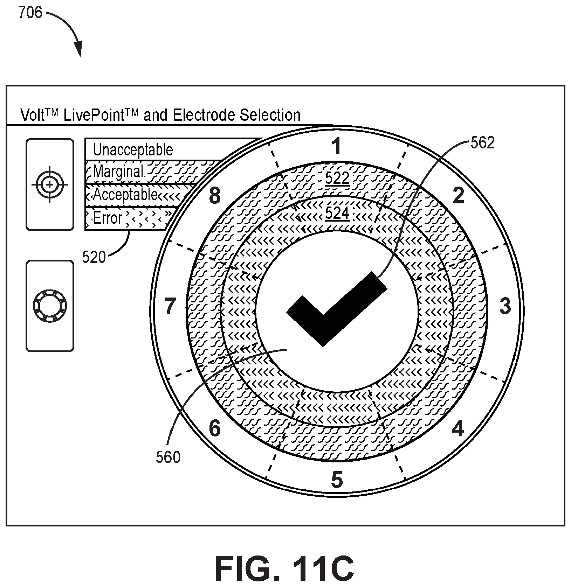

An exemplary embodiment of the baseline collection state 706 is shown in FIGS. 11A-11C. As shown in FIG. 11A the circle sectors 510a-510h each corresponding to an electrode, or an electrode pair (e.g., the electrodes 405a-405h) are initially devoid of any color. As the baseline collection mode progresses and marginal baseline data (see FIG. 11B circle sectors 510c and 510h) and/or acceptable baseline data (see FIG. 11B circle sectors 510a and 510b) is collected, the respective color in the first ring section 522 and/or the second ring section 524 displays a categorized color. Once all eight electrodes or electrode pairs have collected an acceptable baseline measurement, a center circular display 560 may communicate completion of the baseline collection state 706 with a first symbol 562 (see e.g., FIG. 11C).

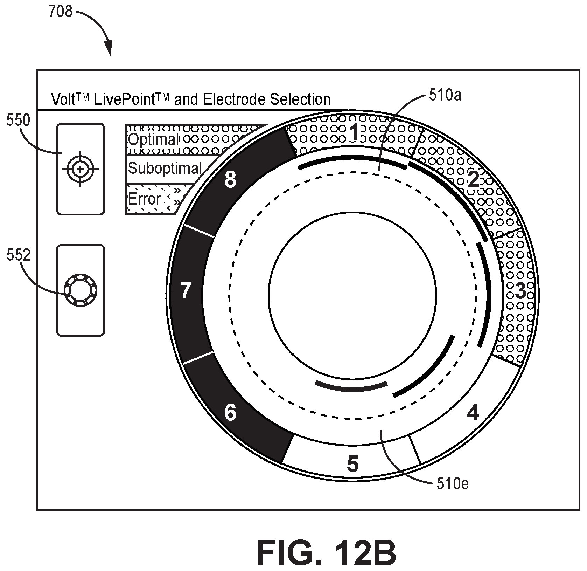

An exemplary embodiment of the tissue proximity state 708 is shown in FIGS. 12A-12C. In the tissue proximity state 708, the electrodes corresponding to each of the circle sectors 510a-510h are selectable, as indicated by the outline area 540. The tissue proximity state 708 displays real-time proximity data on the display 500, with each of the circle sectors 510a-510h including the color area 518a-518h and the graph section 530. FIG. 12A shows the electrodes corresponding to the circle sectors 510a-510e as "active", with circle sector 510e displaying an error value. In some embodiments (such as FIG. 12A), the color area 518 may communicate an "inactive and error" output, wherein the electrode label 516 is assigned a corresponding color (e.g., orange) and the color area 518 is assigned a corresponding color (e.g., dark grey) (see for example, FIG. 12A circle sector 510f). FIG. 12B shows an embodiment of the tissue proximity state 708 where the circle section 510e is no longer displaying an error value (as shown in FIG. 12A).

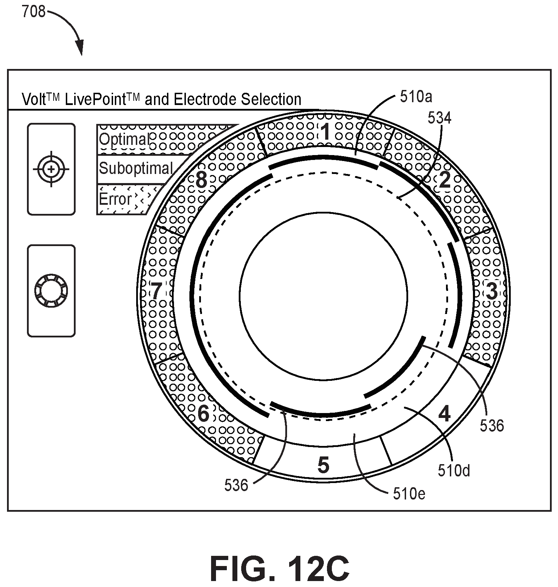

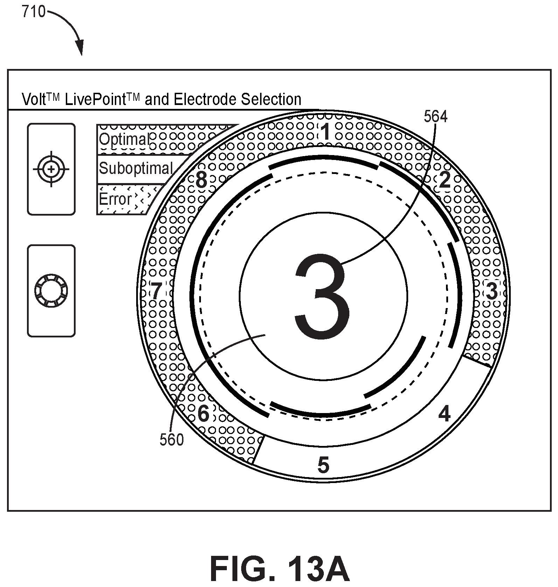

FIG. 12C shows the electrodes corresponding to the circle sectors 510a-510c and 510f-510h as active and optimal, with the circle sectors 510d and 510e as active and suboptimal, each with the current value bar 536 near the threshold line 534. In other words, FIG. 12C illustrates six of eight electrodes in an optimal proximity, with the remaining two electrodes near the optimal proximity. In such cases, the user may choose whether to proceed with catheter manipulation to achieve eight-of-eight optimal values, or whether to proceed with a therapy delivery (see FIGS. 13A-13B).

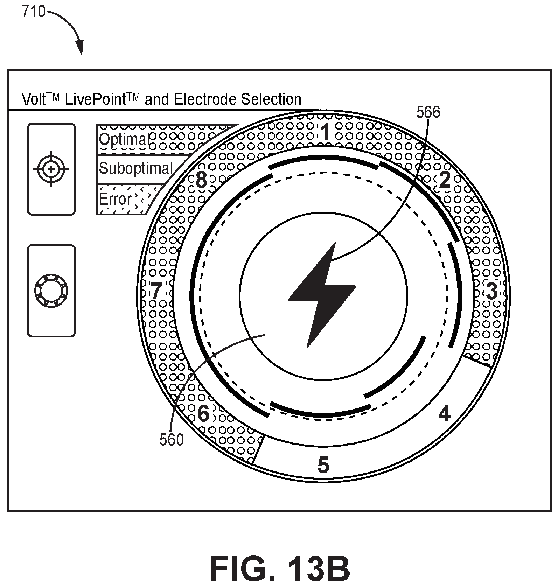

FIGS. 13A-13B illustrate an exemplary embodiment of the therapy delivery state 710. As shown in FIG. 13A, the therapy delivery state 710 may include a countdown 564 displayed in the center circular display 560. The countdown 564 may display a descending time until a therapy is delivered through the catheter device. During the countdown period, the display 500 may continue to display real-time electrode proximity data to the user. The therapy delivery state 710 includes the delivery of a medical treatment through the catheter device. In some embodiments, the medical treatment may include an ablation, a balloon angioplasty, a vascular stent, and/or a drug delivery. In some embodiments, and ablation treatment may be delivered directly through the active electrodes. During the medical treatment, a second symbol 566 may be displayed in the center circular display 560, as shown in FIG. 13B. The display 500 may continue to display real-time electrode proximity data to the user.

In some embodiments, the display user interface 502 may gatekeep functionality of the catheter device. For example, in the tissue proximity state 708 the display user interface 502 may require at least six of the eight electrodes or electrode pairs to have an "optimal" proximity value before the therapy delivery state 710 may begin. In such example, the therapy delivery control (not shown) will not be displayed and/or able to be manipulated until the minimum threshold is met.

In some embodiments, the display user interface 502 may gatekeep functionality of each of the system states 702, 704, 706, 708, and 710. In other words, certain conditions must be met at each system state in order to unlock functionality of the display user interface 502. For example, in the electrode selection state 704, the display user interface 502 may not allow the user to proceed directly to the tissue proximity state 708. Instead, the display user interface 502 may require the user to proceed to the baseline collection state 706 before proceeding to the tissue proximity state 708.

The gatekeeping functionality of the display user interface 502 is beneficial, as it may guide the user, step-by-step through a standardized process. The gatekeeping functionality may help ensure that no steps are skipped and/or that each step is completed to at least a minimum level of satisfaction. Each step and each minimum level of satisfaction may be programed into the ECU 118.

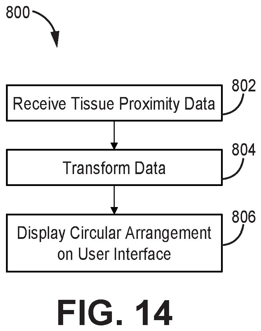

FIG. 14 shows a method of display 800 that includes the display 500, the display user interface 502, and the plurality of circle sectors 510. The method of display 800 includes the following steps: STEP 802, RECEIVE TISSUE PROXIMITY DATA, includes receiving tissue proximity data from a plurality of electrodes or electrode pairs disposed on a medical device. In some embodiments, the tissue proximity data may include BECI measurements. STEP 804, TRANSFORM DATA, includes transforming the received data from numerical values into a circular arrangement of categorized values for each of the plurality of electrodes. In some embodiments, STEP 804 further includes analysis of BECI measurements such as the ratio of measured impedance to a baseline measurement. STEP 806, DISPLAY CIRCULAR ARRANGEMENT ON USER INTERFACE, includes displaying the circular arrangement on a user interface.

### Discussion of Possible Embodiments

The following are non-exclusive descriptions of possible embodiments of the present invention.

According to one aspect, a display system is configured to communicate tissue proximity data from a plurality of electrodes includes a display. The display outputs a circular graphical representation. The circular graphical representation includes a plurality of color areas forming a first circular shape. Each of the color areas communicate a categorized value for a respective electrode indicative of tissue proximity for the respective electrode. A plurality of graph areas form a second circular shape. Each of the graph areas communicate a relative value for the respective electrode indicative of the tissue proximity for the respective electrode.

The display system of the preceding paragraph can optionally include, additionally and/or alternatively, any one or more of the following features, configurations and/or additional components.

For example, the display system may optionally be configured such that the display outputs a graphical user interface. The graphical user interface enables direct manipulation of the plurality of electrodes.

The display system may optionally be configured such that the graphical user interface enables direct manipulation of a treatment device.

The display system may optionally be configured such that the graphical user interface performs one or more gatekeeper functions.

The display system may optionally be configured such that the categorized value is indicative of whether a threshold level of electrode contact is met. The relative value is indicative of an electrode contact value, and in some embodiments, the relative value may include a graphical comparison between the electrode contact value and a threshold line.

The display system may optionally be configured such that the circular graphical representation represents a circular electrode distribution on a medical device.

According to one aspect, a user interface system for a catheter device includes a display. The display includes a circular proximity display area having a plurality of circle sectors. Each of the plurality of circle sectors corresponds to one or more electrodes disposed on the catheter device. The user interface system includes one or more control features configured to manipulate the catheter device and/or the one or more electrodes disposed on the catheter device. The one or more control features are configured to select one or more system states.

The user interface system of the preceding paragraph can optionally include, additionally and/or alternatively, any one or more of the following features, configurations and/or additional components.

For example, the user interface system may optionally be configured such that the one or more system states include a tissue proximity state. The tissue proximity state displays a categorized value of an electrode measurement in each of the plurality of circle sectors.

The user interface system may optionally be configured such that the tissue proximity state displays a relative value of an electrode measurement in each of the plurality of circle sectors.

The user interface system may optionally be configured such that a color segment communicates the categorized value of the electrode measurement and a graph segment displays the relative value of the electrode measurement.

The user interface system may optionally be configured such that the one or more system states include a baseline collection state. The baseline collection state displays a plurality of radial sections within each of the circular sectors. Each of the radial sections communicates a categorized value of a baseline measurement.

The user interface system may optionally be configured such that the one or more system states includes a therapy delivery state. The therapy delivery state initiates an ablation treatment though the catheter device.

The user interface system may optionally be configured such that the one or more control features are configured to gatekeep functionality of the catheter device.

The user interface system may optionally be configured such that the one or more control features are configured to select the one or more electrodes. Selection of an electrode selectively enables the electrode for therapy delivery and/or electrode measurement. Deselection of the electrode selectively disables the electrode for therapy delivery and electrode measurement.

According to one aspect, a method of display includes receiving tissue proximity data from a plurality of electrodes. Received data is transformed into a circular arrangement. The circular arrangement includes a plurality of circle sectors. Each of the circle sectors corresponds to one or more electrodes. The circular arrangement is displayed on a user interface. Each of the circle sectors includes a categorized value display and a relative value display.

The method of display of the preceding paragraph can optionally include, additionally and/or alternatively, any one or more of the following features, configurations and/or additional components.

For example, the method of display may optionally be configured such that the categorized value display includes output values. The output values include an optimal proximity, a sub-optimal proximity, and an error state. Each of the output values corresponds to a respective color output.

The method of display may optionally be configured such that the relative value display includes a threshold line and a current proximity line.

The method of display may optionally be configured such that the plurality of electrodes are arranged on a plurality of splines circumferentially spanning around a central axis of a medical device.

The method of display may optionally be configured such that a number of splines included on the medical devices corresponds to a number of circle sectors of the circular arrangement.

The method of display may optionally be configured such that transforming received data into the circular arrangement includes comparing a measured bipolar electrode complex impedance (BECI) measurement to a baseline BECI measurement.

While the invention has been described with reference to an exemplary embodiment(s), it will be understood by those skilled in the art that various changes may be made and equivalents may be substituted for elements thereof without departing from the scope of the invention. In addition, many modifications may be made to adapt a particular situation or material to the teachings of the invention without departing from the essential scope thereof. Therefore, it is intended that the invention not be limited to the particular embodiment(s) disclosed, but that the invention will include all embodiments falling within the scope of the appended claims.

## Claims

1. A user interface system for a catheter device, the user interface system comprising:
a display, including a circular proximity display area, the circular proximity display area including a plurality of circle sectors, each of the plurality of circle sectors corresponding to one or more electrodes disposed on the catheter device; and
one or more control features configured to manipulate the catheter device and/or the one or more electrodes disposed on the catheter device,
wherein the one or more control features are configured to select one or more system states.

2. The user interface system according to claim 1, wherein the one or more system states include a tissue proximity state, wherein the tissue proximity state displays a categorized value of an electrode measurement in each of the plurality of circle sectors.

3. The user interface system according to claim 1, wherein the one or more system states include a baseline collection state, wherein the baseline collection state displays a plurality of radial sections within each of the circular sectors, each of the radial sections communicating a categorized value of a baseline measurement.

4. The user interface system according to claim 1, wherein the one or more system states includes a therapy delivery state, wherein the therapy delivery state initiates an ablation treatment though the catheter device.

5. The user interface system according to claim 1, wherein the one or more control features are configured to gatekeep functionality of the catheter device.

6. The user interface system according to claim 1, wherein the one or more control features are configured to select the one or more electrodes, wherein selection of an electrode selectively enables the electrode for therapy delivery and/or electrode measurement, and wherein deselection of the electrode selectively disables the electrode for therapy delivery and electrode measurement.
